# EUROPÄISCHE PATENTSCHRIFT

(11) **EP 0 499 834 B1**
(45) Veröffentlichungstag und Bekanntmachung des Hinweises auf die Patenterteilung: **04.11.1998**
(21) Anmeldenummer: 92101271.2
(22) Anmeldetag: 27.01.1992
(51) Int. Cl.: C12N 15/30, A61K 39/015, C12P 21/02, C07K 14/445

(54) **Ein Plasmodium falciparum Blutstadien-Antigen, seine Herstellung und Verwendung**
A blood-stage antigen from Plasmodium falciparum, its preparation and use
Un antigène des stades sanguins de Plasmodium falciparum, sa préparation et son utilisation

(30) Priorität: 21.02.1991 DE 4105348
(43) Veröffentlichungstag der Anmeldung: 26.08.1992
(73) Patentinhaber: Chiron Behring Gmbh & Co., 35006 Marburg (DE)
(72) Erfinder: Nolte, Dagmar, W-3550 Marburg (DE); Knapp, Bernhard, W-3550 Marburg-Schröck (DE); Hundt, Erika, W-3550 Marburg-Wehrshausen (DE); Küpper, Hans Dr., W-3550 Marburg (DE)
(74) Vertreter: Hallybone, Huw George

(56) Entgegenhaltungen:
- WO-A-86/04922
- CELL, Band 44, Nr. 5, 14 März 1986, Cell Press, Cambridge, MA (US); J. KOCHAN et al., Seiten 689-696
- MOLECULAR BIOCHEMISTRY & PARASITOLOGY, Band 49, Nr. 2, 1991, Elsevier Science, Amsterdam (NL); D. NOLTE et al., Seiten 253-264

## Beschreibung

Die Erfindung betrifft eine DNA Sequenz, die der eines Glycophorin bindenden Proteins, dem sogenannten GBP 130, hoch homolog ist und deshalb als GBP 130 h bezeichnet wurde. Weiterhin betrifft die Erfindung das Protein GBP 130 h aus Plasmodium falciparum selbst sowie ein Verfahren zu seiner Herstellung mittels rekombinanter DNA Technik. Schließlich betrifft die Erfindung die Verwendung des Proteins GBP 130 h aus Plasmodium falciparum zur Herstellung von Impfstoffen gegen Malaria.

Der Einzeller Plasmodium falciparum - ein Verursacher der Malaria des Menschen - ist ein zum Stamm der Sporozoa gehörender Blutparasit. Als überträger von Mensch zu Mensch dienen ausschließlich Stechmücken der Gattung Anopheles (und zwar nur die Weibchen), die bein Saugen die Parasiten mit dem Blut aufnehmen bzw. abgeben. Bis zwei Wochen nach dem infizierenden Stich der Mücke findet man die Parasiten innerhalb von roten Blutkörperchen. Sie nehmen in ihnen amöboide Formen an, wachsen schnell heran, werden vielkernig und teilen sich dann in eine entsprechende Zahl von Tochterindividuen (sogenannte Merozoiten), die, durch Zerfall des Blutkörperchens freigeworden, sofort frische Blutkörperchen befallen. Dieser als "Schizogonie" bezeichnete Vermehtungsvorgang beansprucht etwa 24 bis 48 h. Er wiederholt sich immer von neuem, bis die Zahl der Parasiten (Schizonten) so groß ist, daß der Wirtskörper mit einem Fieberanfall auf die giftigen Stoffwechsel- und Zerfallsprodukte der Erythrozyten und Schizonten-Restkörper reagiert.

Nach einer gewissen Zeit setzt die Bildung von Geschlechtsformen (Gamogonie) ein, die innerhalb der roten Blutkörperchen beginnt, aber nur im Darm der Mücke zum Abschluß kommen kann. Aus den sich in der Mücke bildenden Geschlechtsformen entwickeln sich nach der Befruchtung schließlich sogenannte "Sporozoite", die von der Leibeshöhle zur Speicheldrüse der Mücke gelangen und dort beim Stich zusammen mit dem gerinnungshemmenden Speichel der Mücke in die Blutbahn des Menschen injiziert werden. Damit schließt sich der Generationszyklus von Plasmodium falciparum.

Bei der Malaria tropica, die von Plasmodium falciparum verursacht wird, beträgt die Inkubationszeit 7 bis 15 Tage, im Mittel 12 Tage. Die Malaria tropica ist die schwerste und gefährlichste Form der Malaria. Sie führt auch häufiger als die übrigen Formen zu atypischen und damit nicht ohne weiteres an Malaria erinnernden Krankheitsformen. Die Prodromalerscheinungen sind ausgeprägter und folgen sich rascher als bei Malaria tertiana und quartana. Der Fieberanstieg erfolgt plötzlich, der Fieberverlauf ist unregelmäßig. Alle Allgemeinerscheinungen sind bei Malaria tropica wesentlich schwerer als bei den übrigen Formen. Die Parasitämie nimmt im Verlauf der Krankheit rasch zu. In Extremfällen können 20 bis 30 % der Erythrozyten befallen sein. Ohne Behandlung entwikkelt sich in Kürze ein lebensbedrohliches Krankheitsbild mit Hepatomegalie, Bewußtseinsstörungen, hämolytischer Anämie und Leukozytose.

Seit 1956 hat die Weltgesundheitsorganisation der Vereinten Nationen eine weltweite Malariabekämpfung mit teilweise großen Erfolgen, aber auch großen Rückschlägen, organisiert. Die Bemühungen der Weltgesundheitsorganisation machen deutlich, wie erheblich die mit dem Auftreten von Malaria verbundenen Probleme für die Weltbevölkerung sind. Die Bekämpfung der Malaria erfolgte großenteils auf zwei Schienen, nämlich der Bekämpfung des Vektors und Wirtes von Plasmodium falciparum - der Anopheles-Mücke - und andererseits der Entwicklung von Medikamenten zur Behandlung von malariainfizierten personen bzw. Personen, die sich einem erhöhten Infektionsrisiko aussetzen mußten.

Die Bekämpfung der Anopheles-Mücke durch chemische Mittel wie z. B. DDT hat nur teilweise Erfolg gebracht, weil die Mücken in relativ kurzer Zeit Resistenzen gegen die chemischen Bekämpfungsmittel entwickelt haben.

Mit einem ähnlichen Resistenzproblem wurde man konfrontiert, als an verschiedenartige Medikamente zur Prophylaxe und Bekämpfung von Plasmodium falciparum und anderen Plasmodium-Arten entwickelt hatte. Nicht alle im menschlichen Organismus vorkommenden Entwicklungsstadien des Plasmodiums sprechen auf die gleichen Medikamente an. Letztere müssen deshalb, gemäß ihrem Wirkungsnechanismus, in verschiedene Gruppen unterteilt werden:

| Wirkung von Antimalariamitteln auf verschiedene Entwicklungsstadien von Plasmodien | | | | |
|---|---|---|---|---|
| Medikamentengruppe | Asexuelle Blutstadien | Gewebsformen | Gametozyten | Sporozoiten |
| Chinin | ++ | - | + (P.vivax, P.malariae) | - |
| 4-Aminoquinolin | ++ | - | (+) | - |
| Folsäureantagonisten | + | + | + | - |
| 8-Aminoquinolin | ± | ++ | + | - |
| Sulfonamid | + | ? | + | - |
| 9-Chinolinmethanol (Mefloquin) Lariam^{R} | ++ | ? | + | - |

Die prophylaktischen Maßnahmen bestehen ebenfalls einerseits in einer Bekämpfung der Stechmücke, andererseits in Chemoprophylaxe. Für die Chemoprophylaxe können alle zur Therapie verwendeten Mittel eingesetzt werden.

Im Lauf der Zeit haben sich allerdings gegen alle bewährten Mittel Resistenzerscheinungen von Seiten der Plasmodien bemerkbar gemacht. Ein weiterer Nachteil der medikamentösen Therapie bzw. Prophylaxe besteht in den erheblichen Nebenwirkungen, die die verwendeten Chemikalien im menschlichen Körper bewirken. Ebenfalls nachteilig ist, daß eine Prophylaxe bereits mehrere Wochen vor und nach einem möglichen Kontakt mit Plasnodium-befallenen Mücken erfolgen muß, um eine einigermaßen erfolgreiche Abwehr von Malaria zu gewährleisten.

In neuerer Zeit ist deshalb eine andere Möglichkeit der Malariabekämpfung weltweit diskutiert worden. Dabei handelt es sich um die Idee einer Impfung gegen den Malaria-Erreger. Ein erheblicher Forschungsaufwand ist deshalb betrieben worden, um Antigene, die zur Entwicklung eines Impfstoffs gegen das asexuelle Blutstadium von Plasmodium falciparum geeignet sind, zu identifizieren. Eine Möglichkeit wurde darin gesehen, Antigene auf der Oberfläche der Merozoiten cder der infizierten Erythrozyten zu lokalisieren. Genetische Informationen für parasitencodierte oberflächenlokalisierte Antigene der infizierten Wirtszelle, die als Carrier oder Rezeptor ("Cytoadherence", "rosetting") funktionieren könnten, sind bis heute noch nicht isoliert worden. Im Gegensatz dazu wurden Gene für die auf der Merozoitenoberfläche lokalisierten Antigene (MSA I, MSA II) bereits isoliert und detailliert beschrieben (1).

Ein weiteres, auf der Merozoitenoberfläche lokalisiertes Antigen bindet an Glycophorin (2). Glycophorin ist ein Sialoglykoprotein auf der Oberfläche von Erythrozyten. Das Glycophorin bindende Protein (GBP 130), das sich auf der Oberfläche der Merozoiten befindet, ist wahrscheinlich für die Erkennung der Erythrozyten durch die Merozoiten mitverantwortlich und steuert in noch unbekannter Weise die Invasion von Merozoiten in die Erythrozyten (2). GBP 130 ist ein thermostabiles und lösliches Protein, das im Trophozoit- und Schizonten-Stadium synthetisiert wird. Es wird in das Erythrozyten-Cytoplasma transportiert (3); (4); (5). GBP 130 wird zum Zeitpunkt der Schizonten-Freisetzung in vitro in den Kulturüberstand abgegeben. Dabei wurde gezeigt, daß nur eine ganz geringe Fraktion des GBP 130 mit dem Merozoiten schwach assoziiert bleibt ((3), (5)). GBP 130 scheint stattdessen nach Freisetzung an die Erythrozytenmembran, und zwar an das Glycophorin (2), zu binden.

Antikörper mit Spezifität für GBP 130 können die Invasion von Merozoiten in Erythrozyten in vitro hemmen (6). GBP 130 wurde ebenfalls von einer anderen Gruppe (5) als ein 96 kDa-Antigen mit thermoresistenten Eigenschaften beschrieben. GBP 130 wird durch Antiseren, die aus durch medikamentenkontrollierte Infektion vom immunisierten Saimiri-Affen gewonnen wurden, erkannt. Diese Seren erlauben einen Schutz nach passivem Transfer von Affe zu Affe (7); (8)). Die Impfung der Saimiri-Affen mit einer Proteinfraktion, die GBP 130 enthält, führte zu schützender Immunität. Die Seren der so geschützten Affen zeigen dabei eine starke Reaktion mit der 96 kDa-Bande (9). Weiterhin konnte gezeigt werden, daß Antikörper gegen GBP 130 ausschließlich in Seren von immunen Erwachsenen auftraten, jedoch nicht in Seren von Kindern oder Erwachsenen, die ihre Immunität bereits verloren hatten (9).

Das für GBP 130 codierende Gen, das auch als Ag 78 oder 96 tR bezeichnet wird, wurde von drei unterschiedlichen Plasmodium falciparum-Stämmen isoliert ((4), (5), (6)). Es codiert für ein hochkonserviertes Antigen.

Die von der DNA-Sequenz abgeleitete Aminosäuresequenz besteht aus einer geladenen N-terminalen Region aus 225 Aminosäuren, gefolgt von 11 hochkonservierten Wiederholungen (repeats) von 50 Aminosäuren. Das Gen enthält ein kleines Intron, das die Sequenz, die für die mögliche Signalsequenz codiert, unterbricht (10)).

Aus dem vorgenannten Stand der Technik ergibt sich die Aufgabe, weitere Strukturen bzw. Antigene aufzufinden, die im weitesten Sinne an der Wirt-Parasit-Interaktion von Plasmodium mit menschlichen Zellen beteiligt sein könnten. Solche Antigene sind möglicherweise in der Lage, eine schützende Immunität des Menschen gegenüber Plasmodium falciparum und damit gegenüber Malaria zu erzeugen.

Die Aufgabe wurde durch das Auffinden einer DNA mit einer in Seq. ID No 1 bezeichneten Sequenz, die für das dem GBP 130 homologen Protein GBP 130 h codiert, gelöst.

Das als GBP 130 h bezeichnete Antigen besitzt große homologe Bereiche zum bereits bekannten GBP 130. Beide Antigene wurden von den Erfindern im Hinblick auf die Genstruktur, die Genlokalisation und auf konservierte Strukturen bei verschiedenen Parasiten-Stämmen untersucht.

Die Erfindung umfaßt alle DNA-Sequenzen, die mit der in Seq. ID No 1 gezeigten DNA-Sequenz hybridisieren und gleichzeitig für das Protein GBP 130 h codieren.

Gegenstand der vorliegenden Erfindung ist ebenfalls das Protein GBP 130 h sowie ein Verfahren zu seiner Herstellung mittels rekombinanter DNA-Technik.

Schließlich umfaßt die Erfindung die Verwendung des Proteins GBP 130 h zur Herstellung eines Arzneimittels gegen Plasmodium falciparum, wobei dieses Arzneimittel bevorzugt ein Impfstoff ist.

### Identifizierung von lambda-gt11 Klonen, die für GBP 130 h codieren

Eine genomische Plasmodium falciparum EcoRI*-Bibliothek wurde mit einem ³²P-markierten, nichtredundanten 39mer Oligonucleotid gescreent, das von einem synthetischen Peptid abgeleitet wurde, welches zur N-terminalen Proteinsequenz eines möglichen 55 kDa Oberflächen-Antigens korrespondiert. Die Impfung von Aotus-Affen mit diesem synthetischen Peptid in Kombination mit anderen Proteinen führte zu einer schützenden Immunität gegenüber einer Plasmodium falciparum Infektion (11). Das Oligonucleotid, das zum Testen verwendet wurde, entspricht dem "Codon Usage" von Plasmodium falciparum, entsprechend (12). Acht verschiedene Phagenklone wurden isoliert. Die Sequenzierung ihrer integrierten DNA zeigte, daß keines für die N-terminale Sequenz des 55 kDa-Antigens codierte, die in (11) bestimmt wurde. Eine Computeranalyse mit Hilfe des "best fit"-Programms von UWGCG (University of Wisconsin, Genetic Computer Group) zeigte jedoch, daß einer der Phagenklone, nämlich Pfa55-1, ein 1433 bp langes DNA-Segment enthält, das eine Homologie zu einer Sequenz zeigt, die für die C-terminale Region des Glycophorin bindenden Proteins GBP 130 (6) codiert. Das von diesem Segment (Insert) codierte Protein wurde deshalb als GBP 130 homologes Protein, nämlich als GBP 130 h, bezeichnet.

### Isolierung des vollständigen GBP 130 h Gens

Das insertierte DNA-Fragment des Plasmids p55-1/RI* repräsentiert Teile eines Introns, gefolgt von einem Exon mit einem TAA-Stop Codon und 261 bp der 3' nicht codierenden Region (Seq. ID No 1). Für die Isolierung eines 5' überlappenden Subklons wurde die Methode der inversen Polymerase-Kettenreaktion (13) verwendet. Ausgehend von einem genomischen, für GBP 130 h spezifischen Sau3AI Fragment von 1,25 kb wurde eine DNA-Sequenz amplifiziert, die die 5'-Region des DNA-Fragments von Plasmid p55-1/RI* um 985 bp verlängert (Seq. ID No 1). Beide DNA-Fragmente repräsentieren die vollständige codierende Region und 5' und 3' nicht-codierende Sequenzen des GBP 130 h Gens.

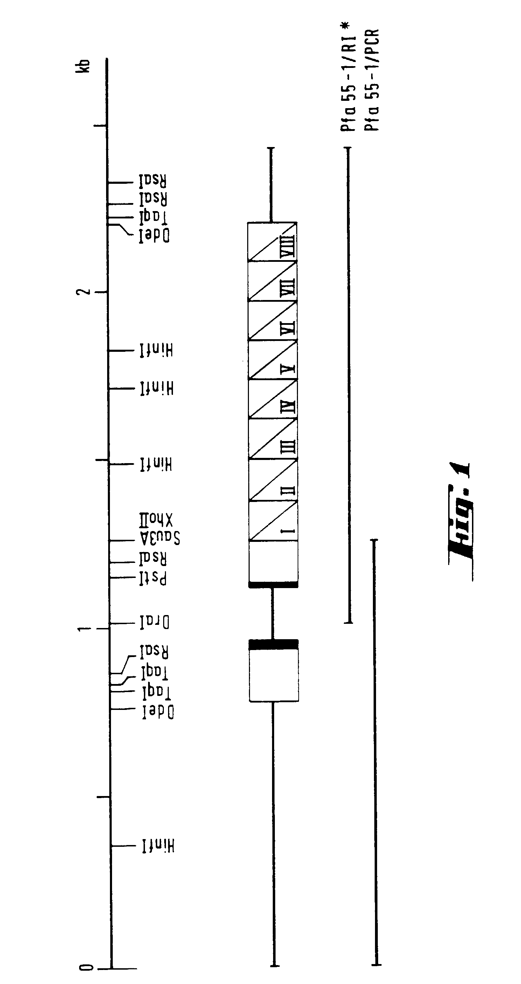

Die in Seq. ID No 1 aufgelistete Sequenz zeigt neben der Nukleotid-Sequenz des vollständigen GBP 130 h Gens des Plasmodium falciparum Stammes FCBR die von der DNA-Sequenz abgeleitete Aminosäuresequenz von GBP 130 h. Fig. 1 zeigt eine Restriktionskarte sowie die Struktur des GBP 130 h Gens. Die codierenden Regionen (boxes) sind durch eine Intron-Sequenz voneinander getrennt dargestellt. Die schwarzen Bereiche korrespondieren zur vorgeschlagenen Signalsequenz. Die Positionen der acht Wiederholungseinheiten (repeat units) sind beschrieben.

Tab. 1 zeigt einen Vergleich der Aminosäuresequenz von GBP 130 h mit der Aminosäuresequenz von GBP 130. Dieser Vergleich wurde mit Hilfe des GAP-Programms von UWGCG vorgenommen. Identität ist gekennzeichnet durch Linien zwischen den entsprechenden Aminosäuren und konservierte Aminosäuresubstitutionen sind durch Doppelpunkte gekennzeichnet.

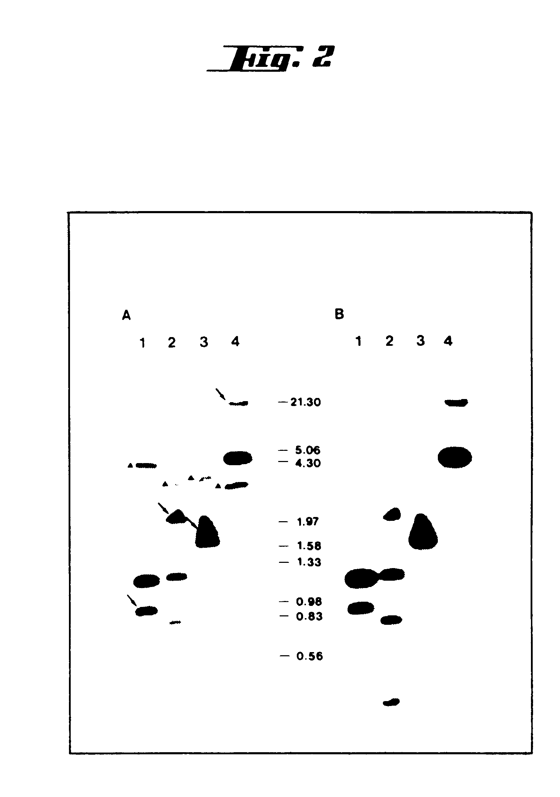

Fig. 2 zeigt eine Southern Blot Analyse von P. falciparum DNA, die mit den Restriktionsenzymen RsaI, HinfI, DraI und EcoRI/XbaI verdaut wurde und mit einem ³²P-markierten XhoII-TaqI Fragment, das die repetitive Region des GBP 130 h Gens beinhaltet, hybridisiert wurde. Der Filter wurde dabei unter milden (A) und stringenten (B) Bedingungen gewaschen. Dabei wurden GBP 130 (Dreiecke)- und GBP 130 h-spezifische DNA-Fragmente sowie DNA-Fragmente eines dritten Gens (Pfeile), welches zu GBP 130 h eine größere Homologie zeigt als zu GBP 130, detektiert.

Die 5' (Nukleotide 1-766) und die 3' (Nukleotide 2202-2418) nicht-codierenden Regionen des GBP 130 h Gens sind extrem A+T reich (89 % bzw. 80,5 %). Dies wurde bereits für nicht-codierende Regionen von anderen Plasmodium falciparum Genen beschrieben (14). Das 155 bp lange Intron (Nukleotide 956-1110) zeigt ebenfalls einen ähnlich hohen A+T Gehalt von 88 %.

Für das GBP 130 Gen (10) wurde an der entsprechenden Position eine intervenierende Sequenz von 179 bp beschrieben, die die Region für die wahrscheinliche Signal-Sequenz unterbricht. Beide Introns beginnen mit GT und enden mit AG und sind somit in Übereinstimmung mit Introns anderer Eukaryonten (15). Die Nukleotidsequenzen beider Introns zeigen eine Homologie von 81 %. Dies zeigt, daß beide Gene in sehr naher Beziehung stehen und daß sie deshalb von einem gemeinsamen Vorläufergen abstammen.

Die beiden Exons des GBP 130 h Gens codieren für 427 Aminosäuren mit einem berechneten Molekulargewicht von 48 260 Da. Das ATG Startcodon befindet sich an Position 767 und ist stromaufwärts flankiert von 4 Adeninresten. Dies ist ebenfalls in Einklang mit den Startkonsensus-Sequenzen von anderen Plasmodium falciparum Genen (16). Der N-Terminus von GBP 130 h beginnt mit einer sehr hydrophilen Region von 50 Aminosäuren, wobei Lysin, Serin und Asparagin sehr häufig vorkommen. Diese Struktur wurde ebenfalls bei GBP 130 gefunden (5), (6). Dieser Region folgt eine hydrophobe Sequenz von 13 Aminosäuren, die von dem 3'-Ende des ersten Exons codiert werden. Diese Region, die zwischen GBP 130 und GBP 130 h hochkonserviert ist, fungiert wahrscheinlich als Signal-Sequenz zusammen mit den folgenden sechs Aminosäuren, die von dem zweiten Exon codiert werden. Die vorausgesagte Signal-Peptidase-Schnittstelle von GBP 130 ist Glycin 69 (6). An der zu dieser Stelle korrespondierenden Position wurde bei GBP 130 h ein Alaninrest gefunden.

Der C-Terminus von GBP 130 h besteht aus einer ausgedehnten wiederholungs-Region (extended repeat region), die 74,5 % des gesamten Proteins repräsentiert. Diese Region enthält acht Wiederholungseinheiten mit 40 Aminosäuren, wobei diese Struktur sehr charakteristisch für GBP 130 h ist. Die Wiederholungen zeigen lediglich geringe Variationen, wobei zwei dieser Wiederholungen, nämlich IV und V lediglich aus 39 Aminosäuren bestehen. Die letzten vier Aminosäuren DELE der Wiederholungen I, II, VI und VII sind durch Nukleotide codiert, die komplementär zu den letzten zwölf Basen des Oligonukleotids sind, das für das Screening benutzt wurde.

Wie durch den Vergleich der Aminosäuresequenz von GBP 130 h und GBP 130 gezeigt wurde, besteht eine Identität von 69 % zwischen den beiden Sequenzen. Dies entspricht einer sehr hohen Homologie. Der Hauptunterschied betrifft ein stark geladenes Segment von 116 Aminosäuren von Position 110 bis 225 in GBP 130, wobei dieses Segment in GBP 130 h nicht vorkommt. Die ersten 46 Aminosäuren, die durch das zweite Exon codiert werden, zeigen eine Identität von lediglich 54 % zwischen beiden Proteinen, womit dieses Segment die am meisten divergierende Region zwischen GBP 130 und GBP 130 h ist. Weiterhin unterscheiden sich die Proteine in der Anzahl und der Länge der Wiederholungen. GBP 130 enthält elf Wiederholungen von 50 Aminosäuren, während GBP 130 h nur acht Wiederholungen mit 40 Aminosäureresten zeigt, welche mit den Aminosäuren 2 bis 41 der GBP 130 Wiederholungen korrespondieren.

### Konservierung des GBP 130 h Gens

DNA-Fragmente, die zu den Nukleotid-Positionen 767 bis 1232 korrespondieren, welche das Exon 1 und das Exon 2 stromaufwärts von den Wiederholungsregionen sowie die intervenierende Sequenz des GBP 130 h Gens enthalten, wurden amplifiziert und dann sequenziert. Dabei handelte es sich um DNA der Plasmodium falciparum Stämme FCBR, FCR-3, SGE2, ItG₂G₁, FVOR, FU1 und #13. Diese 465 bp Region des GBP 130 h Gens ist identisch bezüglich ihrer Sequenz für alle Parasiten-Isolate, die bisher analysiert wurden. Dies zeigt, daß das GBP 130 h Gen hochkonserviert ist.

### GBP 130 h und GBP 130 sind durch verschiedene Gene codiert

Durch PCR an einer genomischen DNA des Plasmodium falciparum Stammes FCBR mit Hilfe der Oligonukleotide p5 und p6 (Tabelle) konnte ein 360 bp langes Fragment, das für die GBP 130 spezifische hochgeladene Region codiert, isoliert werden. Verglichen mit der GBP 130 Sequenz des Stammes FCR-3 (6) zeigt dieses Fragment zwei Basenpaar-Austausche, die in einer Substitution von Aminosäuren resultieren: In Position 713 von GBP 130 ist ein A durch ein C ersetzt und in Position 758 ist ein A durch ein G ersetzt. Dieser Basenaustausch in Position 713 von GBP 130 wurde auch für das Palo Alto Isolat berichtet (10).

Diese GBP 130 spezifische Probe und ein 108 bp PstI-XhoII DNA-Fragment von GBP 130 h (vgl. Fig. 1) wurden für die Southern Blot Analyse von Plasmodium falciparum DNA, geschnitten mit verschiedenen Restriktionsenzymen, verwendet. Beide Proben hybridisierten mit verschiedenen DNA-Fragmenten eines Plasmodium falciparum Stammes. Dies zeigt eindeutig, daß das Genom von Plasmodium falciparum zwei verschiedene Gene für GBP 130 bzw. GBP 130 h enthält.

### Das GBP 130 Gen spezifiziert eine Genfamilie von drei verschiedenen Genen

Die repetitive Region des GBP 130 h Gens wurde isoliert und als Probe zur Southern Blot Analyse von mit den Restriktionsenzymen RsaI, HinfI, DraI und EcoRI/XbaI verdauter genomischer P. falciparum DNA eingesetzt. Unter milden Waschbedingungen (55°C, 2XSSC, 0,1 % SDS) können drei verschiedene Gene detektiert werden. Anhand der bekannten Restriktionskarten des GBP 130 Gens (4, 5, 6) und des GBP 130 h Gens (Fig. 1) sowie mit Hilfe einer Southern Blot Analyse mit GBP 130 und GBP 130 h spezifischen DNA-Fragmenten (351 bp XbaI-SpeI Fragment bei GBP 130, 108 bp PstI-XhoII Fragment bei GBP 130 h), die unter stringenten Bedingungen durchgeführt wurde, konnten die GBP 130 und GBP 130 h spezifischen Hybridisierungsfragmente eindeutig zugeordnet werden. (Fig. 2A) Darüber hinaus konnte ein drittes Gen nachgewiesen werden, wobei mit der GBP 130 h Probe ein ca. 22kb EcoRI/XbaI Fragment, ein 1,7 kb DraI Fragment, ein 0,8 kb RsaI Fragment sowie ein 2,2 kb HinfI-Fragment kreuzhybridisieren. Wird der Filter unter stringenteren Bedingungen (65°C; 0,5XSSC, 0,1 % SDS) gewaschen, so ist keine Hybridisierung der GBP 130 h Probe mit dem GBP 130 Gen zu erkennen, wohl aber werden die DNA-Fragmente, die dem dritten noch unbekannten Gen zugeordnet werden, detektiert (Fig. 2B). Dies zeigt, daß dieses Gen zu dem GBP 130 h Gen homologer ist als zu dem GBP 130 Gen.

### Das GBP 130 h Gen wird in Blutstadien von P. falciparum nur sehr schwach exprimiert

Ausgehend von Poly (A)⁺ RNA aus Schizonten wurde eine Northern Blot Analyse mit einem 108 bp PstI-XhoII Fragment (spezifisches GBP 130 h Gen-Fragment) und einem 351 bp XbaI-SpeI Fragment (spezifisches GBP 130 Gen-Fragment) durchgeführt. Beide zur Hybridisierung eingesetzten Fragmente verfügen über etwa gleiche spezifische Radioaktivität. Die GBP 130 Probe detektierte eine singuläre mRNA von ca. 6,5 kb als dominante Bande nach einer Über-Nacht-Exposition. Ähnliche Ergebnisse für GBP 130 sind bereits in der Literatur beschrieben worden (4, 5, 6). Im Gegensatz dazu hybridisierte die GBP 130 h Probe mit zwei mRNA Banden von ca. 2,5 kb und ca. 2,8 kb, die nur nach sehr langer Expositionszeit von 8 Tagen nachweisbar waren. Dabei kann eine der mRNAs dem GBP 130 h Gen zugeordnet werden; die zweite Bande könnte die mRNA des dritten Gens der GBP-Genfamilie repräsentieren, welches zum GBP 130 h Gen offensichtlich eine hohe Homologie zeigt (Fig. 2). Auffällig ist die Diskrepanz der Expressionsrate des GBP 130 Gens zum Gen, das für das Protin GBP 130 h codiert: Das GBP 130 Gen wird in sehr hoher Effizienz transkribiert, wogegen die Transkriptionsrate des GBP 130 h Gens und offensichtlich des dritten Gens dieser Genfamilie sehr schwach ist. Geht man davon aus, daß die von diesen mRNAs translatierten Proteine etwa ähnliche Häufigkeitsraten aufzeigen, so muß angenommen werden, daß in Schizonten von P. falcipa rum GBP 130 sehr häufig vorkommt und GBP 130 h eher unterrepräsentiert ist. Diese unterschiedliche Verteilung dieser homologen Proteine könnte für den sog. "smoke screen"-Effekt ausgenutzt werden: Dabei wird GBP 130 nach Freisetzung der Merozoiten aus den Schizonten in hoher Menge abgegeben und könnte die Aufgabe haben, das Immunsystem abzulenken, wobei gleichzeitig das unterrepräsentierte GBP 130 h seiner essentiellen Funktion nachgehen könnte.

Die Tabelle 2 zeigt die Sequenzen von Oligonukleotiden, die für die Polymerase-Kettenreaktionen (polymerase chain reaction, PCR) konstruiert wurden.

### Beispiele

### Präparation von DNA und mRNA

Zur Kultivierung der Plasmodium falciparum Stämme FCBR (Kolumbien), FCR-3 (Kolumbien), FVOR (Vietnam), SGE2 (Zaire), ItG₂G₁ (Brasilien), FU1 (Uganda) und #13 (Senegal), für die Anreicherung von Schizonten sowie für die Präparation von DNA und Poly(A)⁺ RNA wurden Standardmethoden verwendet (17). Die Analyse von DNA und mRNA des Stammes FCBR durch Southern und Northern Blot Technologie ist ebenfalls im Stand der Technik beschrieben (18).

### Konstruktion einer genomischen EcoRI*-Bibliothek

2 µg DNA des Plasmodium falciparum Stammes FCBR wurden über Nacht bei 37°C mit 14 Einheiten des Restriktionsenzyms EcoRI in 10 mmol Tris-HCL (pH 7,5), 10 mmol MgCl₂, 1 mmol Dithiotreitol und 40 % (v/v) Glycerol inkubiert. Unter diesen Bedingungen zeigt EcoRI "star"-Aktivität. Dies bedeutet, daß die DNA an ihren Tetranucleotiden AATT verdaut wird. Die dabei entstehenden DNA-Fragmente mit einer Größe bis zu 10 kb wurden mittels eines 0,8 % Agarosegels fraktioniert. Fragmente zwischen 500 bp und 7 kb wurden elektroeluiert und danach in den Vektor lambda gt11 nach der in (19) beschriebenen Methode eingeführt. Eine genomische EcoRI*-Bibliothek von 5 x 10⁵ rekombinanten Phagenklonen wurde dadurch erhalten und diese Phagen wurden dann mit Standardmethoden amplifiziert (20).

### Screening der EcoRI*-Bibliothek

1,5 x 10⁵ Phagenklone dieser Bibliothek wurden mit einem 5' ³²P-markierten Oligonukleotid, das von der N-terminalen Proteinsequenz eines möglichen 55 kDa Plasmodium falciparum Oberflächenantigens abgeleitet wurde (11) nach Standardmethoden gescreent (20). Das Oligonukleotid hatte die Basensequenz: 5'-TGC TGC ATA TAC ATT TTG TGT TTC TGC TTC TAA TTC ATC-3'.

Das Oligonukleotid wurde nach den von Plasmodium falciparum am häufigsten verwendeten Codons (16) konstruiert. Acht Phagenklone wurden isoliert und einer von diesen, der als Pfa55-1 bezeichnet wurde, wurde für die weiteren Untersuchungen verwendet. Die DNA des Phagenklons Pfa55-1 wurde mit den Restriktionsenzymen EcoRI und KpnI verdaut. Dabei wurde ein 2,4 kb Fragment erhalten, das neben dem Malaria-spezifischen Fragment eine 1 kb Region von Lambda gt11 trug, die mit Hilfe einer partiellen PvuII Restriktion entfernt wurde. Das dadurch erhaltene 1,4 kb Fragment wurde in den pKS(+) Bluescript Vektor kloniert, der mit EcoRI und SmaI verdaut wurde, wobei das Plasmid p55-1/RI* entstand.

### Isolierung eines 5' überlappenden Genfragments durch inverse PCR

Um das vollständige Gen zu isolieren, von dem ein Teilstück in dem Phagenklon Pfa55-1 enthalten ist, wurde die 5'-Genregion mit Hilfe von inverser PCR verlängert (13). Ein genomisches 1,25 kb Sau3AI-Fragment, das die 5'-Region der insertierten DNA des Phagenklons Pfa55-1 um 985 bp verlängert, wurde mit Hilfe der Southern Blot-Analyse (20) identifiziert, wobei ein ³²P-markiertes 108 bp Fragment (PstI/XhoII-Verdau von p55-1/RI∗) als Probe diente. 90 µg Sau3AI verdaute P. falciparum DNA wurde auf einem 0,8 % Agarosegel aufgetrennt und die DNA-Fragmente mit einer Größe zwischen 1,2 und 1,3 kb wurden elektroeluiert. Die Sau3AI-Schnittstellen wurden selbstligiert und nach Restriktion mit dem Enzym PstI wurden die bekannten DNA-Sequenzen zum 5'- und 3'-Ende konvertiert. 50 ng dieser genomischen DNA und 500 ng der Oligonukleotide p1 und p2 (Tab. 2) wurden für die PCR verwendet, die unter Standardbedingungen mit Hilfe des Gene-Amp^{R}-Kits von Perkin Elmer Cetus durchgeführt wurde. Das danach erhaltene 1,25 kb Fragment wurde an den 5'-Enden phosphoryliert. Dem folgte eine "fill-in"-Reaktion mit dem Klenow-Enzym (20). Dieses DNA-Fragment wurde dann in den pKS-Vektor, der mit SmaI verdaut worden war, eingeführt. Das so gebildete Plasmid wurde als p55-1/PCR bezeichnet.

### DNA-Sequenzierung

Beide Stränge der insertierten DNA-Fragmente der Plasmide p55-I/RI* und p55-1/PCR wurden mit Hilfe der Dideoxy-Methode sequenziert, wobei das Sequenase-System von USB (Cleveland, OH) verwendet wurde. Geeignete Subfragmente wurden durch Subklonierung an zur Verfügung stehenden Restriktionsstellen in den Bluescript-Vektor pKS erhalten. Die Analyse der Sequenzierungsdaten wurde mit Hilfe des UWGCG-Programms durchgeführt (21).

### Amplifikation und Sequenzierung von spezifischen Genregionen verschiedener P. falciparum-Isolate

0,5 µg DNA der P. falciparum Stämme FCBR, FCR-3, SGE2, ItG₂G₁, FVOR, FU1 und #13 wurden in Kombination mit jeweils 300 ng der Oligonukleotide p3 und p4 (Tab. 2) verwendet, um ein genomisches Fragment zu amplifizieren. Dabei wurde der Gen-Amp^{R}-Kit von Perkin Elmer Cetus verwendet. Die genomischen Fragmente der sieben verschiedenen Parasiten-Isolate wurden phosphoryliert, dann einer "fill-in"-Reaktion mit Hilfe des Klenow-Enzyms nach Standard-Methoden (20) unterworfen und danach in eine SmaI-Schnittstelle des Vektors pKS zur Sequenzierung eingefügt.

### Konstruktion einer GBP 130 spezifischen Probe

Der Vergleich der codierenden Sequenzen von GBP 130 h und GBP 130 (6) führt zur Auffindung eines 351 bp langen Fragments, das nur im GBP 130 Gen vorhanden ist. Dieses GBP 130 spezifische Fragment wurde von einer genomischen DNA des P. falciparum-Stammes FCBR amplifiziert und zwar mit Hilfe der Oligonukleotide p5 und p6 (Tab. 2). Das daraus resultierende 360 bp Fragment wurde mit XbaI und SpeI verdaut und danach in den Vektor pKS ligiert, wobei das Plasmid pKS/GBP entstand. Die Identität des GBP 130 spezifischen Fragments wurde durch DNA-Sequenzierung kontrolliert.

### Southern Blot Analyse des GBP 130 und GBP 130 h Gens

Das GBP 130 spezifische Fragment wurde vom Plasmid pKS/GBP mit Hilfe der Restriktionsenzyme XbaI und SpeI isoliert und durch Nick-Translation mit ³²P markiert. Analog wurde vom Plasmid p55-I/RI* mit Hilfe der Restriktionsenzyme PstI und XhoII ein 108 bp großes GBP 130 h spezifisches DNA-Fragment isoliert und durch Nick-Translation mit ³²P markiert. Beide Proben wurden zur Southern Blot Analyse nach Standard-Methoden (20) von P. falciparum DNA verwendet, die mit den Restriktionsenzymen DdeI, TaqI, AluI, Sau3AI, RsaI, HinfI, DraI und EcoRI/XbaI verdaut wurde.

### Southern Blot Analyse genomischer P. falciparum DNA mit Hilfe der repetitiven GBP 130 h Probe

Das Plasmid p55-1/RI∗ wurde mit Hilfe der Restriktionsenzyme XhoII und TaqI verdaut und ein 965 bp DNA-Fragment, das die 8 Wiederholungseinheiten des GBP 130 h Gens enthält, konnte isoliert werden. Dieses DNA-Fragment wurde mit ³²P durch Nick-Translation (20) radioaktiv markiert und zur Southern Blot Analyse von P. falciparum DNA verwendet, die mit den Restriktionsenzymen RsaI, HinfI, DraI und EcoRI/XbaI verdaut wurde. Die Membran wurde nach der Hybridisierung, die nach Standardbedingungen erfolgte (20), zunächst in 2XSSC (2xSSC ist 300 mM NaCl, 30 mM Nacitrat), 0,1 % SDS (Natriumdodecylsulfat) bei 55°C zweimal für je 15 Minuten gewaschen und anschließend für 3 h autoradiographiert. Nach der Entwicklung des Autoradiogramms wurde die Membran unter stringenteren Bedingungen in 0,5XSSC, 0,1 % SDS bei 65°C zweimal je 15 Minuten gewaschen und anschließend über Nacht exponiert.

### Northern Blot Analyse

Je 10 µg einer Poly(A)⁺ RNA, die vom P. falciparum Stamm FCBR aus Schizonten isoliert wurde, wurden mit Hilfe eines 0,8 % Agarose-Formaldehydgels fraktioniert, danach auf eine Gen-Screen Membran (Du Pont) nach dem Protokoll des Anbieters transferiert und mit einem "nick"-translatierten 108 bp langen PstI-XhoII Fragment des GBP 130 h Gens, das vom Plasmid p55-1/RI* erhalten wurde, und mit einem 351 bp XbaI-SpeI Fragment des GBP 130 Gens, das vom Plasmid pKS/GBP erhalten wurde, hybridisiert. Der Filter wurde bei 55°C zweimal 15 min. in 0,5XSSC, 0,1 % SDS gewaschen und autoradiographiert.

### Expression einer partiellen GBP 130 h Sequenz

Der Vektor pSEM (Biotechniques 8, Seiten 280-281 (22)) wurde für die Expression einer Teilsequenz des Plasmids p55-1/RI* benutzt, wobei das Fusionsprotein die N-terminalen 375 Aminosäuren von β-Galaktosidase trägt. Die Oligonukleotide p7 und p8 (Tabelle) und 100 ng der Plasmid-DNA von p55-1/RI* wurden zur Amplifikation eines 680 bp Fragments mit Hilfe der PCR verwendet. Das amplifizierte Fragment wurde mit SacI und PstI verdaut und danach in den mit den gleichen Restriktionsenzymen linearisierten pSEM1 Vektor ligiert. Der E. coli Stamm DH5alpha wurde mit dem ligierten Plasmid transformiert. Danach wurden Kolonien isoliert, die insertierte DNA-Fragmente der korrekten Größe enthielten. Einzelne Kolonien wurden über Nacht kultiviert und danach mit 1 mM IPTG (Isopropylthiogalactosid) 2 h lang induziert. Die Analyse der Expressionsprodukte wurde mit Hilfe der SDS-Polyacrylamidgelektrophorese durchgeführt. Dabei konnte ein Fusionsprotein von 70 kDa in hoher Ausbeute exprimiert werden.
1. Kemp, D.J., Cowman, A.F. and Walliker, D. (1990) Genetic diversity in Plasmodium falciparum. Advances in Parasitology 29, 75-149.
2. Perkins, M. E. (1984) Surface Proteins of Plasmodium falciparum. Merozoites Binding to the Erythrocyte Receptor, Glycophorin. J. Exp. Med. 160, 788-798.
3. Perkins, M. E. (1988) Stage-Dependent Processing and Localization of a Plasmodium falciparum Protein of 130,000 Molecular Weight. Exper. Parasitol. 65, 61-68.
4. Bianco, A. E., Culvenor, J. G., Coppel, R. L., Crewther, P. E., McIntyre, P., Favaloro, J. M., Brown, G. V., Kemp, D. J. and Anders, R. F. (1987). Putative glycophorin-binding protein is secreted from schizonts of Plasmodium falciparum. Mol. Biochem. Parasitology 23, 91-102.
5. Bonnefoy, S., Mattei, D., Dubremetz, J.-F., Guillotte, M., Jouin, H., Ozaki, L. S., Sibilli, L. and Mercereau-Puijalon, O. (1988) Plasmodium falciparum: Molecular Analysis of a Putative Protective Antigen, the Thermostable 96 kDa Protein. Exper. Parasitology 65, 69-83.
6. Kochan, J., Perkins, M. E. and Ravetch, J. V. (1986) A Tandemly Repeated Sequence Determines the Binding Domain for an Erythrocyte Receptor Binding Protein of Plasmodium falciparum. Cell 44, 689-696.
7. Gysin, J., Dubois, P. and Pereira da Silva, L. (1982) Protective antibodies against erythrocytic stages of Plasmodium falciparum in the experimental infection of the squirrel monkey Saimiri sciureus. Parasite Immunology 4, 421-430.
8. Jouin, H., Dubois, P., Gysin, J., Fandeur, T., Mercereau-Puijalon, O. and Pereira da Silva, L. (1987) Characterization of a 96 kDa thermostable polypeptide antigen of Plasmodium falciparum related to protective immunity. Infect. and Immunity 55, 1287-1392.
9. Dubois, P., Druilhe, P., Arriat, D., Jendoubi, M. and Jouin, H. (1987) Changes in recognition of Plasmodium falciparum antigens by human sera depending on previous malaria exposure. Ann. l'Institut Pasteur, Immunologie (Paris) 138, 383-396.
10. Bonnefoy, S. and Mercereau-Puijalon, O. (1989) Plasmodium falciparum: An Intervening Sequence in the GBP 130/96 tR Gene. Exp. Parasitol. 69, 37-43.
11. Patarroyo, M. E., Romero, P., Torres, M. L., Clavijo, P., Moreno, A., Martinez, A., Rodriguez, R., Guzman, F. and Cabezas, E. (1987) Induction of protective immunity against experimental infection with malaria using synthetic peptides. Nature 328, 629-632.
12. Hyde, J. E., Kelly, S. L., Holloway, S. P., Snewin, V.A. and Sims, P. F. G. (1989) A general approach to isolating Plasmodium falciparum genes using non-redundant oligonucleotides inferred from protein sequences of other organisms. Mol. Biochem. Parasitol. 82, 247-262.
13. Triglia, T., Peterson, M. G. and Kemp, D. J. (1988) A procedure for in vitro amplification of DNA segments that lie outside the boundaries of known sequences. Nucl. Acids Res. 16, 8186.
14. Weber, J. L. (1987) Analysis of sequences from the extremely A+T-rich genome of Plasmodium falciparum. Gene 52, 103-109.
15. Mount, S. M. (1982) A catalogue of splice junction sequences. Nucl. Acids. Res. 10, 459-472.
16. Saul, A. and Battistutta, D. (1990) Analysis of the sequences flanking the translational start sites of Plasmodium falciparum. Mol. Biochem. Parasitol. 42, 55-62.
17. Knapp, B., Shaw, A., Hundt, E., Enders, B. and Küpper, H. A. (1988) A histidine-alanine-rich recombinant antigen protects Aotus monkeys from Plasmodium falcinarum infection. Behring Res. Commun. 82, 349-359.
18. Knapp, B., Hundt, E., Nau, U. and Küpper, H. A. (1989) Molecular cloning, genomic structure and localization of a blood stage antigen of Plasmodium falciparum characterized by a serine stretch. Mol. Biochem. Parasitol. 32, 73-84.
19. Huynh, T. V., Young, R. A. and Davis, R. W. (1985) Constructing and screening cDNA libraries in lambdagt10 and lambdagt11 (in: DNA-cloning Vol. 1 pp. 49-78 ed. by Glover, D. M.).
20. Sambrook, J., Fritsch, E. F. and Maniatis, T. (1989) Molecular Cloning: A Laboratory Manual. 2nd edn., Cold Spring Harbor Laboratory, Cold Spring Harbor, NY.
21. Devereux, J.; Haeberli, P. and Smithies, O. (1984) A comprehensive set of sequence analysis programs for the VAX. Nucl. Acids Res. 12, 387-395
22. Knapp, S., Bröker, M. and Amann, E. (1990) pSEM Vectors: High Level Expression of Antigenic Determinants and Protein Domains. Biotechniques 8, 280-281.

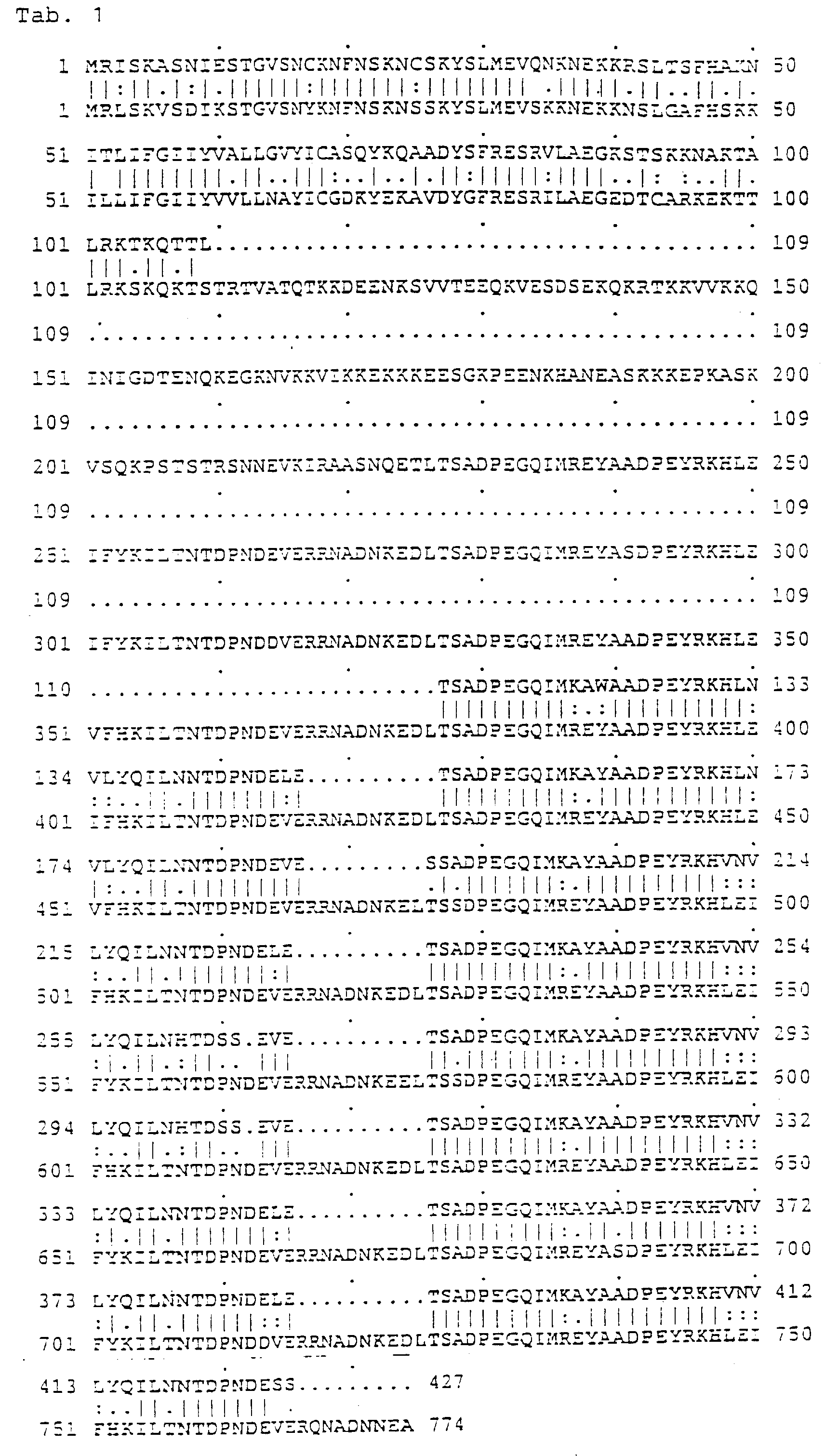

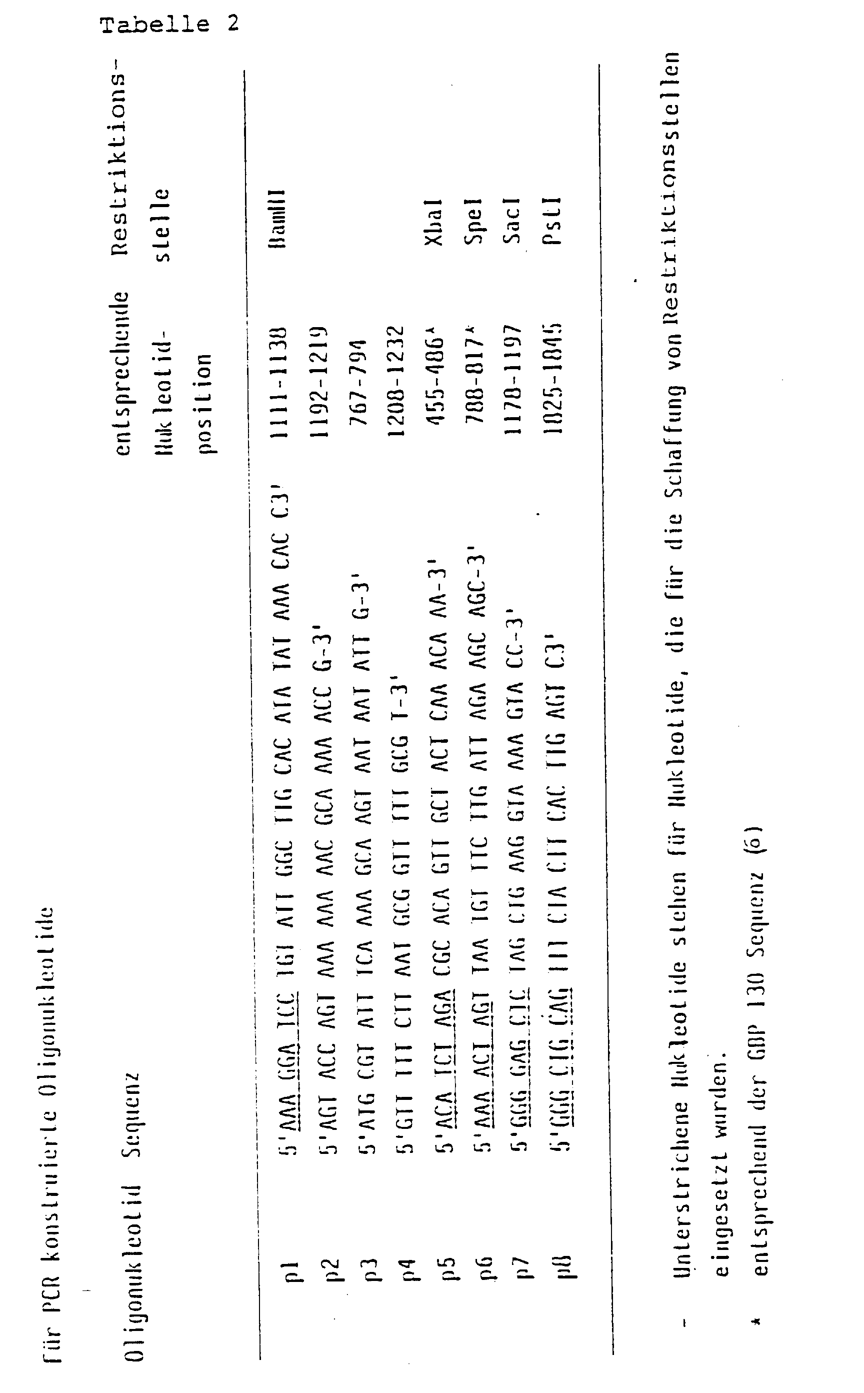

SEQ ID NO: 1
ART DER SEQUENZ: Nukleotid mit entsprechenden Protein
STRANGFORM: Einzelstrang
TOPOLOGIE: linear
ART DES MOLEKÜLS: Genom-DNA
URSPRÜNGLICHE HERKUNFT
ORGANISMUS: Plasmodium falciparum
UNMITTELBARE EXPERIMENTELLE HERKUNFT
NAME DES STAMMES: P. falciparum FCBR
MERKMALE:
von 767 bis 955 BP Exon1
von 1111 bis 2202 BP Exon2
von 1249 bis 2202 BP repetitive Region
EIGENSCHAFTEN:Gen, da für ein dem GBP130 Protein homologes Antigen codiert

GATCTATATT AAAAAAAATA TACAAGGAAA AGATGTGTTA AACAATATAC ATTTATATAA 60

TATATAATTA ATATAATATA ATATAATGTA GTATTTCATG AAATATATTA TGTAGGTTTG 120

ATTTAAATTT AATCATTATA TAAATATTAC ACATATGAAT AAATAAATAA ATATATATAT 180

ATATATATAA ATATATTTAC TTATATTTAG TAATTTTTAA CATTGTATAT TTAAAAAGAA 240

AATATTTTTT ATTTCATATT TTTTAATATT TTATAATAGA AAAAGATAGA TACAAAAGAA 300

CATGTGGAAA AAATATATAT TATAAAATAT ATTCTAAATT CTTTTATTAA TTTTAAGATT 360

CAATAAAAAT AATATATCAA TGAAATAATT AATTATTTTA TAATCAATAG TCGTAAGTGT 420

AATACATATA TTATTTCTAG CTTCTTGTAA CTTACATAAT TATAAATATA TCATATATTC 480

TTTCAAGGAT ATGATAATTT ATATCATTGA AAAAATATAT ATATAGTATT TATCTTTTAT 540

GAAAAAAAAC ATTGAAATGT AATTTATGTA AAAAAAAAAA AAATTAAAAT AAAATAATAA 600

AAAAATATTT ATGTATTGTT TTTTTTTTTT ATTTTTATTT TATTATTTTA AAATATATAT 660

TTAAAAAAAA AAAAATATAT ATATATATAA TATTTATATT TATAATTATT TTAGAAACAC 720

ACAAATTAGA AAAAACATAT ATATTCTTAT TTTCTTCTAA GTAAAA ATG CGT ATT 775
Met Arg Ile

TCA AAA GCA AGT AAT ATT GAA TCT ACA GGA GTT TCG AAT TGT AAA AAT 823
Ser Lys Ala Ser Asn Ile Glu Ser Thr Gly Val Ser Asn Cys Lys Asn
    5                   10                  15

TTC AAT TCG AAA AAT TGC TCT AAA TAT TCT TTG ATG GAA GTA CAA AAT 871
Phe Asn Ser Lys Asn Cys Ser Lys Tyr Ser Leu Met Glu Val Gln Asn
20                  25                  30                  35

AAA AAT GAA AAG AAA CGT TCC TTA ACT TCT TTC CAT GCC AAA AAC ATC 919
Lys Asn Glu Lys Lys Arg Ser Leu Thr Ser Phe His Ala Lys Asn Ile
                40                  45                  50

ACA TTG ATT TTT GGA ATA ATA TAC GTA GCC TTA TTG GTATGA TAATATAAAT 971
Thr Leu Ile Phe Gly Ile Ile Tyr Val Ala Leu Leu
            55                  60

AAAATATATA ATTGAATTTT TTTCTTTTTT AATTAATGAA TTTAAATATC CTTATTAGAA 1031

ATATATAGAT ACATAGATAC ATACATAAAT ATATATTTAT ATATATATAT TTTTTTCTGT 1091

TTGCATCATT TATTTTTAG GGT GTT TAT ATA TGT GCA AGC CAA TAC AAA CAA 1143
Gly Val Tyr Ile Cys Ala Ser Gln Tyr Lys Gln
    65                  70

GCT GCA GAT TAT AGT TTT AGA GAA AGC AGA GTT TTA GCT GAA GGT AAA 1191
Ala Ala Asp Tyr Ser Phe Arg Glu Ser Arg Val Leu Ala Glu Gly Lys
75                  80                  85                  90

AGT ACC AGT AAA AAA AAC GCA AAA ACC GCA TTA AGA AAA ACT AAG CAA 1239
Ser Thr Ser Lys Lys Asn Ala Lys Thr Ala Leu Arg Lys Thr Lys Gln
                95                  100                 105

ACA ACC TTA ACT AGC GCA GAT CCA GAA GGA CAA ATA ATG AAA GCC TGG 1287
Thr Thr Leu Thr Ser Ala Asp Pro Glu Gly Gln Ile Met Lys Ala Trp
            110                 115                 120

GCT GCT GAT CCA GAA TAT CGT AAA CAC CTA AAT GTT CTT TAC CAA ATA 1335
Ala Ala Asp Pro Glu Tyr Arg Lys His Leu Asn Val Leu Tyr Gln Ile
        125                 130                 135

TTA AAT AAC ACT GAT CCA AAT GAT GAA TTA GAA ACT AGC GCT GAC CCA 1383
Leu Asn Asn Thr Asp Pro Asn Asp Glu Leu Glu Thr Ser Ala Asp Pro
    140                 145                 150

GAA GGA CAA ATA ATG AAA GCT TAT GCT GCT GAT CCA GAA TAT CGT AAA 1431
Glu Gly Gln Ile Met Lys Ala Tyr Ala Ala Asp Pro Glu Tyr Arg Lys
155                 160                 165                 170

CAC CTA AAT GTT CTT TAC CAA ATA TTA AAT AAT ACC GAC CCA AAT GAT 1479
His Leu Asn Val Leu Tyr Gln Ile Leu Asn Asn Thr Asp Pro Asn Asp
                175                 180                 185

GAA GTA GAA TCT AGC GCT GAC CCA GAA GGA CAA ATA ATG AAA GCT TAT 1527
Glu Val Glu Ser Ser Ala Asp Pro Glu Gly Gln Ile Met Lys Ala Tyr
            190                 195                 200

GCT GCT GAT CCA GAA TAT CGT AAA CAC GTA AAT GTC CTT TAC CAA ATA 1575
Ala Ala Asp Pro Glu Tyr Arg Lys His Val Asn Val Leu Tyr Gln Ile
        205                 210                 215

TTA AAT AAC ACC GAT CCA AAT GAT GAA TTA GAA ACT AGC GCA GAC CCA 1623
Leu Asn Asn Thr Asp Pro Asn Asp Glu Leu Glu Thr Ser Ala Asp Pro
    220                 225                 230

GAA GGA CAA ATA ATG AAA GCC TAC GCA GCT GAT CCA GAA TAT CGT AAA 1671
Glu Gly Gln Ile Met Lys Ala Tyr Ala Ala Asp Pro Glu Tyr Arg Lys
235                 240                 245                 250

CAT GTA AAT GTC CTT TAC CAA ATA TTA AAT CAC ACC GAC TCA AGT GAA 1719
His Val Asn Val Leu Tyr Gln Ile Leu Asn His Thr Asp Ser Ser Glu
                255                 260                 265

GTA GAA ACT AGC GCA GAC CCA GAA GGA CAA ATA ATG AAA GCT TAT GCT 1767
Val Glu Thr Ser Ala Asp Pro Glu Gly Gln Ile Met Lys Ala Tyr Ala
            270                 275                 280

GCT GAT CCA GAA TAT CGT AAA CAC GTA AAT GTC CTT TAC CAA ATA TTA 1815
Ala Asp Pro Glu Tyr Arg Lys His Val Asn Val Leu Tyr Gln Ile Leu
        285                 290                 295

AAT CAC ACC GAC TCA AGT GAA GTA GAA ACT AGC GCA GAC CCA GAA GGA 1863
Asn His Thr Asp Ser Ser Glu Val Glu Thr Ser Ala Asp Pro Glu Gly
    300                 305                 310

CAA ATA ATG AAA GCC TAC GCA GCT GAT CCA GAA TAT CGT AAA CAC GTA 1911
Gln Ile Met Lys Ala Tyr Ala Ala Asp Pro Glu Tyr Arg Lys His Val
315                 320                 325                 330

AAT GTC CTT TAT CAA ATA TTA AAT AAC ACT GAT CCA AAT GAT GAA TTA 1959
Asn Val Leu Tyr Gln Ile Leu Asn Asn Thr Asp Pro Asn Asp Glu Leu
                335                 340                 345

GAA ACC AGC GCA GAC CCA GAA GGA CAA ATA ATG AAA GCT TAT GCA GCT 2007
Glu Thr Ser Ala Asp Pro Glu Gly Gln Ile Met Lys Ala Tyr Ala Ala
            350                 355                 360

GAT CCA GAA TAT CGT AAA CAC GTA AAT GTC CTT TAT CAA ATA TTA AAT 2055
Asp Pro Glu Tyr Arg Lys His Val Asn Val Leu Tyr Gln Ile Leu Asn
        365                 370                 375

AAC ACT GAT CCA AAT GAT GAA TTA GAA ACT AGT GCT GAC CCA GAA GGA 2103
Asn Thr Asp Pro Asn Asp Glu Leu Glu Thr Ser Ala Asp Pro Glu Gly
    380                 385                 390

CAA ATA ATG AAA GCT TAT GCT GCT GAT CCA GAA TAT CGT AAA CAC GTT 2151
Gln Ile Met Lys Ala Tyr Ala Ala Asp Pro Glu Tyr Arg Lys His Val
395                 400                 405                 410

AAT GTC CTT TAC CAA ATA TTA AAT AAC ACT GAT CCA AAT GAT GAA AGT 2199
Asn Val Leu Tyr Gln Ile Leu Asn Asn Thr Asp Pro Asn Asp Glu Ser
                415                 420                 425

TCC TAAGAA TGTATCTCCC TTCGAAAAAT AAGAGAAAAC AAAATTTGCA AATGAATTAG 2258
Ser

AAAGTACGAT TATGATAATT AAGAGATGTA TGAATTTGAA TGTAAAAATG ACATTTTTTA 2318

TAATAACGTA CAATATTTTA ATAATTAATT ATCAAAAATG AAATATATAA TACTATTTAT 2378

GGTATTTGAT ATTATTTAGA TGAGGAAGAA AAAAGGAATT 2418

## Patentansprüche (Patentansprüche für folgende(n) Vertragsstaat(en): AT, BE, CH, DE, DK, FR, GB, IT, LI, LU, NL, PT, SE)

1. DNA, die für das Protein GBP 130 h gemäß Seq. ID No 1 codiert.

2. DNA, dadurch gekennzeichnet, daß sie mit einer DNA gemäß Anspruch 1 unter stringenten Bedingungen hybridisiert.

3. Protein GBP 130 h mit einer in Seq. ID No 1 bezeichneten Aminosäuresequenz.

4. Protein, dadurch gekennzeichnet, daß es von einer DNA gemäß Anspruch 1 oder 2 codiert wird.

5. Verfahren zur Herstellung eines Proteins gemäß Anspruch 3 oder 4, dadurch gekennzeichnet, daß ein Wirtsorganismus mit einer DNA gemäß Anspruch 1 oder 2 transformiert wird und daß das Protein nach Kultivierung des Wirtsorganismus und Expression isoliert wird.

6. Protein gemäß Anspruch 3 oder 4 als Arzneimittel.

7. Impfstoff, der ein Protein gemäß Anspruch 3 oder 4 enthält.

8. Verwendung eines Proteins gemäß Anspruch 3 oder 4 zur Herstellung eines Arzneimittels gegen Plasmodium falciparum

## Patentansprüche (Patentansprüche für folgende(n) Vertragsstaat(en): ES, GR)

1. Verfahren zur Herstellung eines Proteins, dadurch gekennzeichnet, daß ein Wirtsorganismus mit einer DNA, die für das Protein GBP 130 h gemäß Seq. ID No 1 codiert, oder mit einer DNA, die mit der genannten DNA unter stringenten Bedingungen hybridisiert, transformiert wird und daß das Protein nach Kultivierung des Wirtsorganismus und Expression isoliert wird.

2. Verfahren zur Herstellung eines Proteins nach Anspruch 1 zur Verwendung als Arzneimittel.

3. Verfahren zur Herstellung eines Proteins nach Anspruch 1 zur Verwendung als Impfstoff.

4. Verwendung eines Proteins hergestellt gemäß Anspruch 1 zur Herstellung eines Arzneimittels gegen Plasmodium falciparum.

## Claims (Claims for the following Contracting State(s): AT, BE, CH, DE, DK, FR, GB, IT, LI, LU, NL, PT, SE)

1. DNA which codes for the protein GBP 130 h shown in Seq. ID No. 1.

2. DNA which hybridizes with a DNA as claimed in Claim 1 under stringent conditions.

3. The protein GBP 130 h with an amino-acid sequence indicated in Seq. ID No. 1.

4. A protein which is encoded by a DNA as claimed in Claim 1 or 2.

5. A process for the preparation of a protein as claimed in Claim 3 or 4, which comprises transforming a host organism with a DNA as claimed in Claim 1 or 2, and isolating the protein after cultivation of the host organism and expression.

6. A protein as claimed in Claim 3 or 4 as medicinal agent.

7. A vaccine which comprises a protein as claimed in Claim 3 or 4.

8. The use of a protein as claimed in Claim 3 or 4 for producing a medicinal agent against Plasmodium falciparum.

## Claims (Claims for the following Contracting State(s): ES, GR)

1. A process for the preparation of a protein, which comprises transforming a host organism with a DNA which codes for the protein GBP 130 h shown in Seq. ID No. 1, or with a DNA which hybridizes with said DNA under stringent conditions, and isolating the protein after cultivation of the host organism and expression.

2. The process for the preparation of a protein as claimed in Claim 1 for use as medicinal agent.

3. The process for the preparation of a protein as claimed in Claim 1 for use as vaccine.

4. The use of a protein prepared as claimed in Claim 1 for producing a medicinal agent against Plasmodium falciparum.

## Revendications (Revendications pour l'(les) Etat(s) contractant(s) suivant(s): AT, BE, CH, DE, DK, FR, GB, IT, LI, LU, NL, PT, SE)

1. ADN, codant pour la protéine GBP 130 h selon la séq. ID No 1.

2. ADN, caractérisé en ce qu'il hybride avec un ADN selon la revendication 1 dans des conditions stringentes.

3. Protéine GBP 130 h avec une séquence d'acide aminé spécifiée dans la séq. ID No 1.

4. Protéine, caractérisée en ce qu'elle est codée par un ADN selon la revendication 1 ou 2.

5. Procédé de fabrication d'une protéine selon la revendication 3 ou 4, caractérisé en ce qu'un organisme hôte est transformé avec un ADN selon la revendication 1 ou 2, et en ce que la protéine est isolée après culture de l'organisme hôte et expression.

6. Protéine selon la revendication 3 ou 4 sous forme de médicament.

7. Vaccin, contenant une protéine selon la revendication 3 ou 4.

8. Utilisation d'une protéine selon la revendication 3 ou 4 pour la fabrication d'un médicament contre le plasmodium falciparum.

## Revendications (Revendications pour l'(les) Etat(s) contractant(s) suivant(s): ES,GR)

1. Procédé de fabrication d'une protéine, caractérisé en ce qu'un organisme hôte est transformé avec un ADN, codant pour la protéine GBP 130 h selon la séq. ID No 1, ou avec un ADN, hybridant avec ledit ADN dans des conditions stringentes, et en ce que la protéine est isolée après culture de l'organisme hôte et expression.

2. Procédé de fabrication d'une protéine selon la revendication 1 pour utilisation comme médicament.

3. Procédé de fabrication d'une protéine selon la revendication 1 pour utilisation comme vaccin.

4. Utilisation d'une protéine fabriquée selon la revendication 1 pour la fabrication d'un médicament contre le plasmodium falciparum.
